# EUROPEAN PATENT APPLICATION

(11) **EP 2 407 550 A1**
(43) Date of publication of application: **18.01.2012**
(21) Application number: 10750768.3
(22) Date of filing: 01.03.2010
(51) Int. Cl.: C12Q 1/24, C12N 1/00

(54) **CELL SEPARATION METHOD**

(30) Priority: 10.03.2009 JP 2009056303
(71) Applicant: Sony Corporation, Tokyo 108-0075 (JP)
(72) Inventor: NITTA Nao, TOKYO 108-0075 (JP)
(74) Representative: Körber, Martin Hans
(86) International application number: PCT/JP2010/053702
(87) International publication number: WO 2010/104014

(57) **Abstract**

Provided is a cell separating method which suppresses physical damage to cells without using an apparatus having a complicated structure, and enables the cell separation performed by a quantitative analysis and a multi-parametric analysis and the cell separation using Hoechst staining or the like as an index.

The present invention provides a cell separating method including a step (S3) of determining the characteristics of a cell labeled with a caged compound; a step (S4) of activating the caged compound labeling a target cell by emitting light only to the target cell having a predetermined characteristic; and a step (S5) of bringing cells into contact with a substance having an affinity with the activated caged compound and separating only the target cell from the cells based on the interaction between the substance and the activated caged compound.

## Description

### Technical Field

The present invention relates to a cell separating method. More specifically, the invention relates to a cell separating method in which only a target cell having a predetermined characteristic is separated from a cell population by using a caged compound.

### Background Art

A cell separating technique separating a target cell having a predetermined characteristic from a cell population is widely used for blood cell discrimination, the purification of genetically modified cells, the analysis of changes in cell phenotype, and the like. Examples of the cell separating technique that have been used hitherto include Fluorescence Activated Cell Sorting (FACS) and a method of using magnetic microbeads.

For example, when a target cell expressing a predetermined cell surface antigen is separated using FACS, a cell population including target cells labeled with a fluorescence-labeled antibody which binds specifically to the antigen is caused to flow through a flow cell, followed by laser beam irradiation. Thereafter, the fluorescence emitted from each cell is detected to determine the optical characteristics of the cell, whereby only a target cell having optical characteristics resulting from the labeled fluorescent substance is physically separated. Patent Literature 1 (Fig. 7) discloses FACS including a fluid system in which cells stained with a fluorescence labeling reagent or the like are arranged in a line in the flow cell and discharged out of the flow cell as droplets, an optical system in which the cells are irradiated with a laser beam to detect scattering light and fluorescence, and a sorting system in which the movement direction of the discharged droplets are controlled.

For example, when a target cell expressing a predetermined cell surface antigen is separated using magnetic microbeads, the target cell is labeled with a magnetically labeled antibody which binds specifically to the antigen. Thereafter, the target cells binding to a sufficient amount of the magnetically labeled antibody are captured using a magnet so as to be separated from cells which have not bound to the magnetically labeled antibody (or cells binding to a small amount of the antibody).
Patent Literature 2 discloses a method and apparatus for magnetically separating cells.
Citation List
Patent Literature
[PTL 1] JP-A-2007-46947
[PTL 2] JP-T-2002-515319

### Summary of Invention

According to FACS, it is possible to separate target cells by determining the cell characteristics at a high speed of thousands to tens of thousands of cells per second. However, on the other hand, in order to perform the high speed separation, the cells need to be caused to flow through the flow cell under a high pressure, so physical damage is inflicted on the cells due to the sudden pressure change or the like in a nozzle portion forming droplets. Moreover, the structure of the fluid system and the sorting system of FACS is complicated, and the FACS apparatus is very expensive.

According to the method of using magnetic microbeads, a large amount of cells can be treated collectively, and the physical damage to the cells can be reduced compared to FACS. However, in this method, since the target cell determination cannot be performed by quantitatively analyzing the optical characteristics detected for each cell, for example, it is impossible to separate only the cells expressing the predetermined surface antigen equal to or higher than a certain standard. Moreover, in this method, since a plurality of expression states of surface antigens cannot be analyzed in combination, it is impossible to analyze the cells using multiple parameters and to separate the cells through gating. In addition, in the method of using the magnetic microbeads, since labeling and separation of cells are performed using the magnetically labeled antibody of the cell surface antigen, the cells cannot be separated based on the characteristics of cells that cannot be labeled according to the antibody. Specific examples of the characteristics of cells that cannot be labeled according to the antibody include fluorescence staining of nucleic acid using Hoechst reagent, and fluorescence staining of intracellular calcium using Fluo-3 reagent. A Cell showing high efflux ability with respect to Hoechst reagent is called a "side population cell (SP cell)", and it is mentioned that SP cell has stem cell properties. The SP cell separation using the fluorescence intensity of the cell stained with Hoechst reagent as an index is widely performed in the field of stem cell research, but the SP cell separation cannot be performed by the method of using the magnetic microbeads.

A main object of the invention is to provide a cell separating method which suppresses the physical damage to cells without using an apparatus having a complicated structure, and enables the cell separation performed by quantitative analysis and multi-parametric analysis and the cell separation using Hoechst staining or the like as an index.

In order to solve the above problems, the invention provides a cell separating method including a step of determining the characteristics of a cell labeled with a caged compound; a step of activating the caged compound labeling a target cell by emitting light only to the target cell having a predetermined characteristic; and a step of bringing cells into contact with a substance having an affinity with the activated caged compound and separating only the target cell from the cells based on the interaction between the substance and the activated caged compound.

This cell separating method includes, for example, a step of determining the characteristics of a cell labeled with caged biotin; a step of activating the caged biotin labeling a target cell by emitting light only to the target cell having a predetermined characteristic; and a step of bringing cells into contact with streptavidin and separating only the target cell from the cells based on the interaction between the streptavidin and the activated caged biotin.

Alternatively, this cell separating method includes, for example, a step of determining the characteristics of a cell labeled with caged fluorescein; a step of activating the caged fluorescein labeling a target cell by emitting light only to the target cell having a predetermined characteristic; and a step of bringing cells into contact with an anti-fluorescein antibody and separating only the target cell from the cells based on the interaction between the antibody and the activated caged fluorescein.

The invention also provides, as a modified example of the above cell separating method, a cell separating method including a step of determining the characteristics of a cell labeled with a caged compound; a step of activating the caged compound labeling a non-target cell by emitting light only to the non-target cell not having a predetermined characteristic; and a step of bringing cells into contact with a substance having an affinity with the activated caged compound and obtaining a target cell by removing only the non-target cell from the cells through separation based on the interaction between the substance and the activated caged compound.

In these methods, as the substance having an affinity with the activated caged compound, a substance having the affinity which is expressed when a photocleavable protecting group is cleaved from the caged compound is used.

The invention also provides, as a modified example of the above two cell separating methods, a cell separating method including a step of determining the characteristics of a cell labeled with a caged compound; a step of activating the caged compound labeling a non-target cell by emitting light only to the non-target cell having a predetermined characteristic; and a step of bringing cells into contact with a substance having an affinity with a non-activated caged compound and separating only a target cell from the cells based on the interaction between the substance and the non-activated caged compound.

Alternatively, the invention provides a cell separating method including a step of determining the characteristics of a cell labeled with a caged compound; a step of activating the caged compound labeling a target cell by emitting light only to the target cell having a predetermined characteristic; and a step of bringing cells into contact with a substance having an affinity with a non-activated caged compound and obtaining the target cell by removing only a non-target cell from the cells through separation based on the interaction between the substance and the non-activated caged compound.

In these methods, as the substance having an affinity with the non-activated caged compound, a substance having the affinity which is lost when a photocleavable protecting group is cleaved from the caged compound is used.

The cell separating method according to the invention may further include a step of labeling a cell with a caged compound.

In the invention, the "caged compound" refers to a compound in which an active site thereof is protected by a photocleavable (photodegradable) protecting group, which is a compound shifted to an active state from an inactive state when the photocleavable protecting group is cleaved from the active site by light irradiation. In addition, in the invention, a "caged-type compound" refers to an inactive caged compound in which the active site thereof is protected by the photocleavable protecting group, and an "uncaged-type compound" refers to a caged compound activated when the photocleavable protecting group is cleaved due to the light irradiation.

Furthermore, in the invention, the "active state" of the caged compound refers to a state where the caged compound can interact with the "substance having an affinity with the activated caged compound". Contrary to this, the "inactive state" refers to a state where the caged compound cannot interact with the substance. That is, the "substance having an affinity with the activated caged compound" has an affinity only with the "uncaged-type compound" and does not exhibit an affinity with the "caged-type compound". On the other hand, the "substance having an affinity with the non-activated caged compound" has an affinity only with the "caged-type compound" and does not exhibit an affinity with the "uncaged-type compound"

According to the invention, a cell separating method is provided which suppresses the physical damage to cells without using an apparatus having a complicated structure, and enables the cell separation performed by quantitative analysis and multi-parametric analysis and the cell separation using Hoechst staining or the like as an index.

### Brief Description of Drawings

Fig. 1 is a flowchart illustrating the sequence of a cell separating method according to the invention.
Fig. 2 is a schematic view illustrating the sequence of a cell separating method according to a first embodiment of the invention.
Fig. 3 is a schematic view illustrating the sequence of a cell separating method according to the first embodiment of the invention.
Fig. 4 is a schematic view illustrating the sequence of the cell separating method according to a second embodiment of the invention.
Fig. 5 is a schematic view illustrating the sequence of the cell separating method according to the second embodiment of the invention.

### Description of Embodiments

Hereinafter, preferable embodiments of the invention will be described with reference to drawings. Although the embodiments described as follows illustrate an example of representative embodiments of the invention, the scope of the invention is not narrowly interpreted by the embodiments. The description will be made in the following order.

### 1. Cell separation using caged biotin

(1) Caged compound labeling (step S1)
(2) Fluorescent substance labeling (step S2) and characteristic determination (step S3)
(3) Uncaging (step S4)
(4) Separation (step S5)

### 2. Cell separation using caged fluorescein

(1) Caged compound labeling (step S1)
(2) Fluorescent substance labeling (step S2) and characteristic determination (step S3)
(3) Uncaging (step S4)
(4) Separation (step S5)

### 1. Cell separation using caged biotin

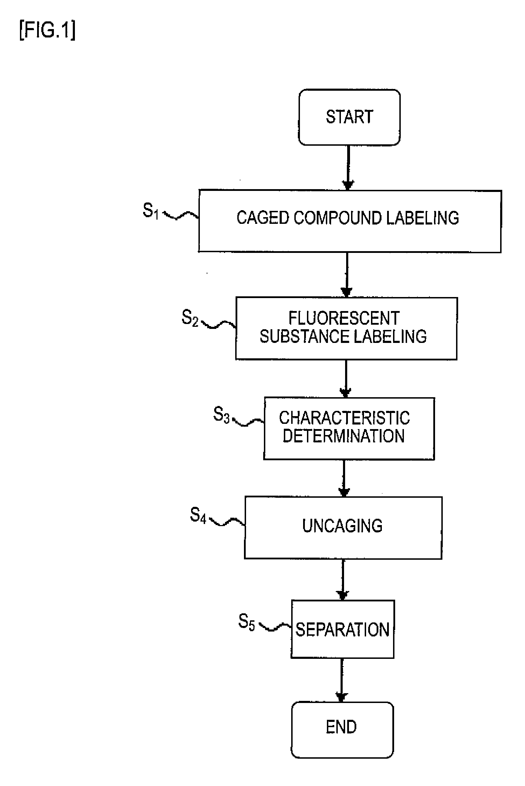

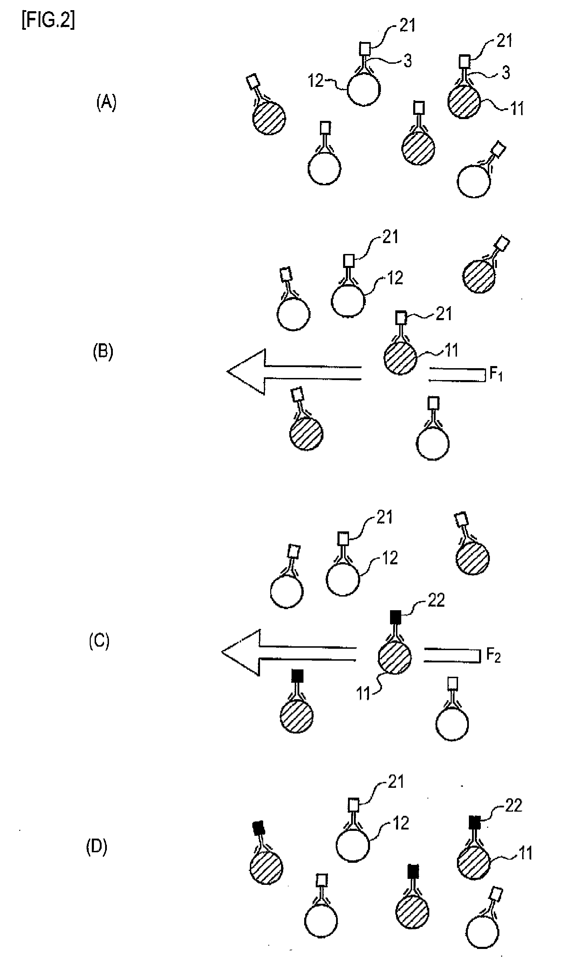

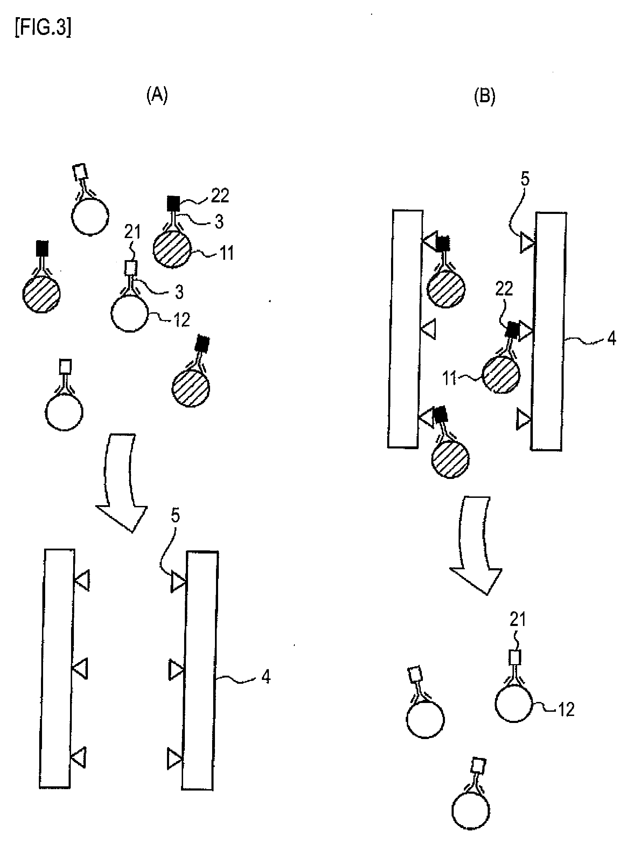

Fig. 1 is the flowchart illustrating the sequence of the cell separating method according to the invention. Figs. 2 and 3 are schematic views illustrating the sequence of the cell separating method according to the first embodiment of the invention. Hereinafter, the sequence of the cell separating method according to the first embodiment will be described with reference to Figs. 1 to 3.

### (1) Caged compound labeling (step S1)

In Fig. 1, the "caged compound labeling step" indicated by reference numeral S1 is a step of labeling a cell with a caged compound (see Fig. 2(A)). In this step, all of the cells including target cells to be separated are labeled with the caged compound.

The cells can be labeled with the caged compound by using, for example, an antibody binding to the caged compound. In this case, as the antigen, the one taking a substance on the surface of all of the cells including the target cells as an antigen is used. Examples of the substance on the surface of all of the cells include cell membrane protein expressed ubiquitously on the cell surface, and a sugar chain as well as lipid existing ubiquitously on the cell surface.

The cells can be labeled with the caged compound by using, for example, lectin binding to the caged compound. In this case, as the lectin, one is used that can bind to the sugar chain existing ubiquitously on the surface of all of the cells including the target cells. In addition, the cells can be labeled with the caged compound using any method without limitation as long as the method enables the cells to directly or indirectly bind to the caged compound.

As the caged compound labeling the cells, for example, caged biotin or caged fluorescein can be used. As the caged compound, caged nucleotide, caged peptide, caged calcium chelate agent, and the like are available, and a caged compound including nucleic acid, protein, lipid, and the like has been reported (see "Biologically active molecules with a "light switch"." Angew Chem Int Ed Engl. 2006 Jul 24;45(30):4900-21). In the invention, any of the above caged compounds can be used without any particular limitation as long as the object of the invention can be achieved.

Fig. 2(A) illustrates a case of labeling target cell 11 and non-target cell 12 with caged-type compound 21 by using antibody 3 in caged compound labeling step S1. Hereinafter, in the embodiment, a case of using caged biotin as caged-type compound 21 will be described for example. As the caged biotin, it is possible to use MeNPOC-biotin, Hydroquinone-biotin, and the like. Herein after, in the embodiment, caged-type compound 21 refers to "caged-type biotin 21".

### (2) Fluorescent substance labeling (step S2) and characteristic determination (step S3)

In Fig. 1, the "characteristic determination step" indicated by reference numeral S3 is a step of determining the characteristics of the cell that has been labeled with the caged compound (see Fig. 2(B)). In this step, the detection and determination of the optical characteristics of the cell that has been labeled with caged-type biotin 21 are performed.

The optical characteristics of the cell can be detected using, for example, a well-known flow cytometer. Specifically, a sample solution including target cell 11 and non-target cell 12 is caused to flow through the flow cell, followed by laser beam F2 irradiation, thereby detecting light to be measured which is generated from the cells. As the light to be measured, forward-scattered light, side-scattered light, scattering light of Rayleigh-scattering, Mie-scattering, or the like, and fluorescence can be used. The detected light to be measured is converted into electric signals, and the characteristics of each cell are determined based on the electric signals.

When the optical characteristics of the cell are determined by detecting the fluorescence emitted from the cells, target cell 11 (or non-target cell 12) may be labeled with a fluorescence-labeled antibody prior to characteristic determination step S3. In Fig. 1, the "fluorescent substance labeling step" indicated by reference numeral S2 is a step of labeling target cell 11 with the fluorescence-labeled antibody binding specifically to the antigen of target cell 11, in a case of separating target cell 11 expressing a predetermined cell surface antigen. Fluorescent substance labeling step S2 can be performed as the same step as the cell separating method using the conventional FACS. Moreover, fluorescent substance labeling step S2 may be performed simultaneously with or prior to caged compound labeling step S1.

In the embodiment, the cell characteristics are detected and determined optically; however, the detection and determination of the cell characteristics may be performed using, for example, an electric or magnetic detector. When the cell characteristics are electrically or magnetically detected, microelectrodes are disposed in the flow cell so as to measure the cells flowing through the flow cell in terms of a resistance value, a capacitance value, an inductance value, impedance, value change in the electric field between electrodes, or, magnetization, the change in magnetic field and the like. In this case, the cells are separated based on the electric or magnetic characteristic of the cell.

### (3) Uncaging (step S4)

In Fig. 1, the "uncaging step" indicated by reference numeral S4 is a step of activating the caged compound labeling the target cell by emitting light only to the target cell having a predetermined characteristic (see Fig. 2(C)). In this step, laser beam F2 is emitted to target cell 11 which has been determined to have a predetermined characteristic in characteristic determination step S3, thereby activating labeling caged-type biotin 21.

As laser beam F2 for activating the caged compound, a laser beam having a wavelength and intensity suitable for cleaving the photocleavable protecting group which protects the active site of the caged compound is used. As the laser source, for example, the same source as that of the above-described laser beam F1 provided in the well known flow cytometer, such as a mercury lamp, a xenon lamp, and various laser beam sources (a solid-state laser, a gas laser, and a semiconductor laser) can be used. As the laser beam source, a giant pulse laser which is small but can emit a high-power laser can be suitably used.

Laser beam F2 for detecting the optical characteristics of the cell and laser beam F2 for activating the caged compound are configured with separate optical systems or the same optical system. In addition, laser beams F1 and F2 can be emitted to target cell 11 at different timings or at substantially the same timing. Herein, the "substantially same timing" means that the emission of laser beam F1 to target cell 11 and non-target cell 12 and the emission of laser beam F2 to target cell 11 that has been determined to have a predetermined characteristic are performed at the same site in the flow cell. By appropriately adjusting the flow speed of the cell in the flow cell according to the time taken for determining the cell characteristic, it is possible to perform the emission of laser beam F1 and laser beam F2 at substantially the same timing.

When laser beam F2 is emitted, caged-type biotin 21 labeling target cell 11 is shifted to the active state from the inactive state due to the cleavage of the photocleavable protecting group from the active site. That is, as shown in Fig. 2(C), caged-type biotin 21 turns into "uncaged-type biotin 22". On the other hand, since laser beam F2 is not emitted to non-target cell 12, caged-type biotin 21 labeling non-target cell 12 is not activated and not changed.

Fig. 2(D) is a view schematically illustrating target cell 11 and non-target cell 12 which have been collected from the flow cell after uncaging step S4. As shown in the drawing, uncaged-type biotin 22 has labeled target cell 11, and caged-type biotin 21 has labeled non-target cell 12.

### (4) Separation (step S5)

In Fig. 1, the "separation step" indicated by reference numeral S5 is a step of bringing the cells into contact with a substance having an affinity with the activated caged compound and separating only the target cell from the cells based on the interaction between the substance and the activated caged compound (see Fig. 3). In this step, target cell 11 labeled with uncaged-type biotin 22 is brought into contact with streptavidin 5 having an affinity with uncaged-type biotin 22. Thereafter, target cell 11 is separated based on the interaction between uncaged-type biotin 22 and streptavidin 5.

Specifically, first, as shown in Fig. 3 (A), target cell 11 and non-target cell 12 collected from the flow cell are caused to flow through column 4 where streptavidin 5 has been made into a solid phase. Target cell 11 labeled with uncaged-type biotin 22 is held in column 4 due to the interaction between uncaged-type biotin 22 and streptavidin 5, as shown in Fig. 3(B). On the other hand, non-target cell 12 labeled with caged-type biotin 21 passes through column 4 without being held in column 4. As a result, it is possible to separate target cell 11 from non-target cell 12. Target cell 11 is not necessarily captured and held completely in column 4 by the interaction between uncaged-type biotin 22 and streptavidin 5. There just may be a time difference in passing through column 4 between target cell 11 and non-target cell 12 passing without interacting with streptavidin 5, by the interaction with streptavidin 5.

Herein, a case has been described in which, in uncaging step S4, laser beam F2 is emitted to target cell 11, and target cell 11 is held in column 4 so as to be separated. However, in the cell separating method according to the embodiment, it is possible to emit laser beam F2 to non-target cell 12 in uncaging step S4. In this case, due to the interaction between activated uncaged-type biotin 22 and streptavidin 5, non-target cell 12 is held in column 4 so as to be separated. Accordingly target cell 11 that has been labeled with caged-type biotin 21 passes through column 4 as is and is collected.

In the embodiment, as column 4, a column in which streptavidin has been made into a solid phase is used. However, any substance can be used as the substance to be made into a solid phase in column 4 as long as the substance has an affinity with uncaged-type biotin 22. Example of the substance includes an anti-biotin antibody. When another compound such as caged fluorescein or the like is used as the caged compound, a substance having an affinity with the uncaged-type compound such as an anti-fluorescein antibody or the like is made into a solid phase in column 4.

As described above, according to the cell separating method of the embodiment, by performing characteristic determination step S3 using the conventional flow cytometer, it is possible to quantitatively and multi-parametrically analyze the optical characteristics of the cells. As a result of the analysis, it is possible to activate only the caged compound of the target cell that has been determined to have a predetermined characteristic and to separate the target cell. According to the cell separating method of the embodiment, it is possible to separate the target cell without using FACS having a complicated structure of the fluid system and the sorting system.

In the cell separating method according to the embodiment, by performing separation step S5 using column 4 after collecting the cells from the flow cell, it is possible to prevent the physical damage to the cells resulting from the droplet formation, which is a problem of separation using FACS. Moreover, when cells having a risk, such as concern over infection, are separated by FACS, there is a problem of contamination caused by aerosol generated during droplet formation. However, in the cell separating method according to the embodiment, there is no concern over the contamination.

### 2. Cell separation using caged fluorescein

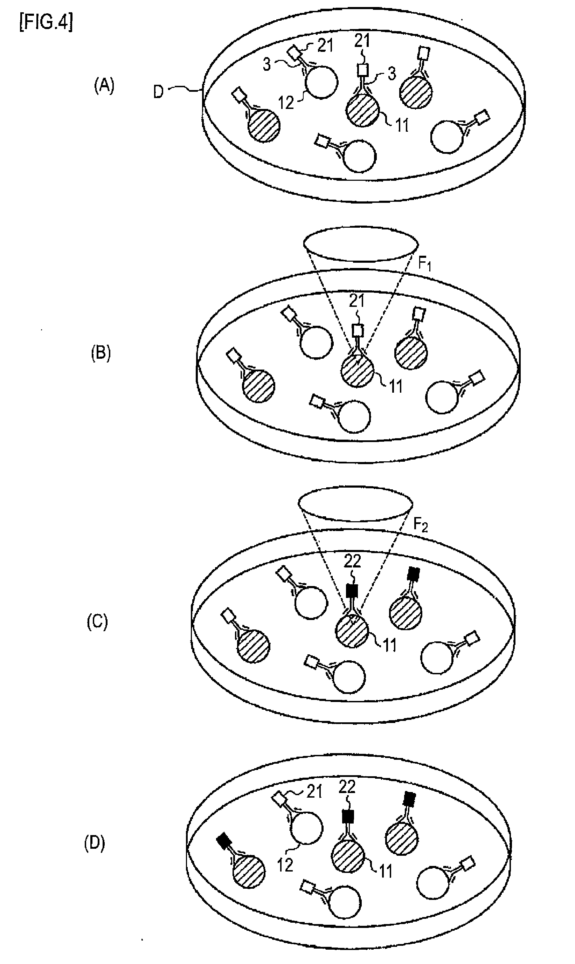

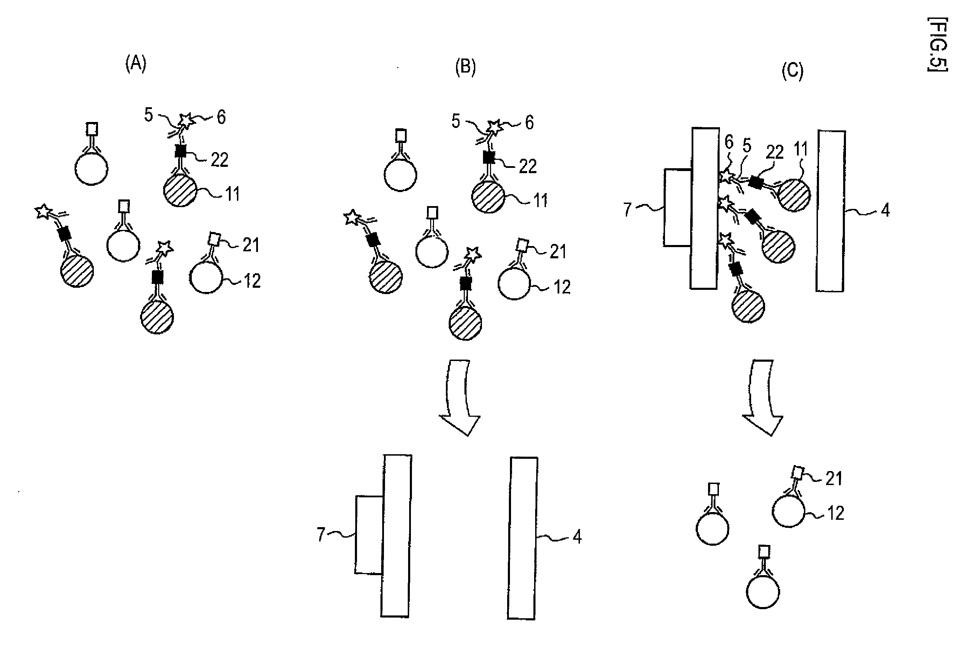

Figs. 4 and 5 are schematic views illustrating the sequence of the cell separating method according to the second embodiment of the invention. In the method according to the first embodiment, characteristic determination step S3 and uncaging step S4 are performed in the flow cell. Contrary to this, the cell separating method according to the present embodiment is **characterized in that** characteristic determination step S3 and uncaging step S4 are performed by means of an imaging technique using a microscope.

### (1) Caged compound labeling (step S1)

Fig. 4(A) schematically shows caged compound labeling step S1 in which target cell 11 and non-target cell 12 which have been stored in container D such as a Petri dish are labeled with the caged compound. In caged compound labeling step S1, all of the cells including the target cells to be separated are labeled with the caged compound.

The cells can be labeled with the caged compound by using, for example, the antibody binding to the caged compound, or lectin binding to the caged compound, as described in the first embodiment. The cells may be floating cells or adherent cells. By adding antibodies or the like in the culture medium in container D where the cells have cultured, or by adding an antibody solution to the culture medium which has been substituted with a proper buffer solution, the caged compound directly or indirectly binds to the cells.

Fig. 4(A) illustrates a case of labeling target cell 11 and non-target cell 12 with caged-type compound 21 by using antibody 3 in caged compound labeling step S1. Hereinafter, in the embodiment, a case of using caged fluorescein as caged-type compound 21 will be described for example. As the caged fluorescein, for example, it is possible to use the one obtained by adding 5-carboxymethoxy-2-nitrobenzyl (CMNB) to fluorescein. Hereinafter, in the embodiment, caged-type compound 21 refers to "caged-type fluorescein 21".

### (2) Fluorescent substance labeling (step S2) and characteristic determination (step S3)

Fig. 4(B) schematically illustrates characteristic determination step S3 in which the optical characteristics of the cells labeled with the caged-type fluorescein 21 is detected and determined.

In the embodiment, the optical characteristics of the cells are detected by, for example, the imaging technique using a microscope. Specifically, for example, by using a laser beam F1 emission device and a microscope as an imaging device including an area imaging device such as CCD or CMOS device, the fluorescence which is generated from the cell due to the emission of laser beam F1 is detected, and the detected fluorescence is converted into electric signals. The characteristics of each cell are determined based on the electric signals. In this case, prior to characteristic determination step S3, target cell 11 (or non-target cell 12) may be labeled with the fluorescence-labeled antibody, in the same manner as described above (see fluorescent substance labeling step S2).

Herein, the characteristics of the cells are determined by detecting the fluorescence. However, the characteristics of cells can also be detected and determined based on the cell shape obtained by the imaging device, for example.

### (3) Uncaging (step S4)

Fig. 4(C) schematically illustrates uncaging step S4 in which laser beam F2 is emitted to target cell 11 that has been determined to have a predetermined characteristic in characteristic determination step S3, whereby labeling caged-type fluorescein 21 is activated.

The emission device of the laser beam F2 is provided in the microscope as a separate or the same optical system. As the laser beam F2 for activating the caged compound, a laser beam having a wavelength and intensity suitable for cleaving the photocleavable protecting group which protects the active site of the caged compound is used. In addition, laser beams F1 and F2 can be emitted to target cell 11 at different timings or at substantially the same timing.

When laser beam F2 is emitted, caged-type fluorescein 21 labeling target cell 11 is shifted to the active state from the inactive state due to the cleavage of the photocleavable protecting group from the active site. That is, as shown in Fig. 4(C), caged-type fluorescein 21 turns into "uncaged-type fluorescein 22". On the other hand, since laser beam F2 is not emitted to non-target cell 12, caged-type fluorescein 21 labeling non-target cell 12 is not activated and not changed.

Fig. 4(D) is a view schematically illustrating target cell 11 and non-target cell 12 after uncaging step S4. As shown in the drawing, uncaged-type fluorescein 22 has labeled target cell 11, and caged-type fluorescein 21 has labeled non-target cell 12.

### (4) Separation (step S5)

Fig. 5 schematically illustrates separation step S5 in which the cells are brought into contact with a substance having an affinity with the activated caged compound, and only the target cells are separated from the cells based on the interaction between the substance and the activated caged compound. In this step, target cell 11 labeled with uncaged-type fluorescein 22 is brought into contact with anti-fluorescein antibody 5 having an affinity with the uncaged-type fluorescein 22. Based on the interaction between the uncaged-type fluorescein 22 and the anti-fluorescein antibody 5, target cell 11 is separated.

Specifically, first, anti-fluorescein antibody 5 binding to magnetic substance 6 is added to target cell 11 and non-target cell 12 which have been collected from container D such as a Petri dish. Anti-fluorescein antibody 5 binds specifically to uncaged-type fluorescein 22 labeling target cell 11, whereby target cell 11 is magnetically labeled with magnetic substance 6 (see Fig. 5(A)). On the other hand, since anti-fluorescein antibody 5 does not bind to non-target cell 12 labeled with caged-type fluorescein 21, non-target cell 12 is not magnetically labeled.

Thereafter, as shown in Fig. 5(B), target cell 11 and non-target cell 12 are caused to flow through column 4 provided with magnet 7. The magnetically labeled target cell 11 is held in column 4 due to the magnetic pulling force acting between magnetic substance 6 and magnet 7 (see Fig. 5(C)). On the other hand, the non-target cell which has not been magnetically labeled passes through column 4 without being held in column 4. As a result, it is possible to separate target cell 11 from non-target cell 12. It is not necessary that target cell 11 is adsorbed onto the inner wall of column 4 and completely held therein by the magnetic pulling force acting between magnetic substance 6 and magnet 7. There just may be a time difference in passing through column between target cell 11 and non-target cell 12 by the magnetic pulling force.

Herein, a case has been described in which, in uncaging step S4, laser beam F2 is emitted to target cell 11, and target cell 11 is held in column 4 so as to be separated. However, in the cell separating method according to the embodiment, it is also possible to emit laser beam F2 to non-target cell 12 in uncaging step S4. In this case, since the activated uncaged-type fluorescein 22 binds to anti-fluorescein antibody 5, non-target cell 12 is magnetically labeled and held in column 4 so as to be separated. Accordingly, target cell 11 which has not been magnetically labeled passes through column 4 so as to be collected.

In the embodiment, caged fluorescein is used as the caged compound, but the caged compound is not limited to caged fluorescein. When a caged compound other than caged fluorescein is used, target cell 11 is magnetically labeled using an antibody binding specifically to the uncaged-type compound instead of anti-fluorescein antibody 5.

As described above, according to the cell separating method of the embodiment, by performing characteristic determination step S3 using the imaging technique, it is possible to quantitatively and multi-parametrically analyze the optical characteristics of cells. As a result of the analysis, it is possible to activate only the caged compound of the target cell determined to have a predetermined characteristic, and to separate the target cell. In addition, according to the cell separating method of the embodiment, it is possible to separate the target cell without using FACS having a complicated structure of the fluid system and sorting system.

According to the cell separating method of the embodiment, by performing separation step S 5 using column 4 after collecting the cells from container D such as a Petri dish, it is possible to prevent the physical damage to the cells caused by the droplet formation, which is a problem caused when separation is performed using FACS. Furthermore, when cells having a risk, such as a concern over infection, are separated by FACS, there is a problem of contamination caused by aerosol generated during the droplet formation. However, the cell separating method according to the embodiment does not have a concern over the contamination.

In the cell separating method according to the first and second embodiments as described above, an example has been described in which the target cell or the non-target cell is separated by using a substance (streptavidin 5 or anti-fluorescein antibody 5) which has a specific affinity not with the caged-type compound but only with the uncaged-type compound. However, in the cell separating method according to the invention, needless to say, it is also possible to separate the target cell or the non-target cell by using a substance which has a specific affinity not with the uncaged-type compound but only with the caged-type compound.

For example, as the anti-fluorescein antibody, an antibody which binds not to uncaged-type fluorescein but only to caged-type fluorescein is prepared. Subsequently, caged-type fluorescein labeling the non-target cell is activated in uncaging step S4, and the target cell labeled with uncaged-type fluorescein is left as it is. As a result, it is possible to hold only the target cell in a column in which the anti-fluorescein antibody has been made into a solid phase and to separate the target cell.

### Industrial Applicability

The cell separating method according to the invention can be widely used for blood cell discrimination, the purification of genetically modified cells, the analysis of the change in cell phenotype, and the like. The cell separating method according to the invention can suppress the physical damage to the cells and enables the cell separation through the quantitative or multi-parametrical analysis. Therefore, the method is useful in a field of hematology or stem cell research.

### Reference Signs List

3: ANTIBODY
4: COLUMN
5: SUBSTANCE HAVING AFFINITY WITH UNCAGED-TYPE COMPOUND 22 (STREPTAVIDIN OR ANTI-FLUORESCEIN ANTIBODY)
6: MAGNETIC SUBSTANCE
7: MAGNET
11: TARGET CELL
12: NON-TARGET CELL
21: CAGED COMPOUND (CAGED-TYPE BIOTIN OR CAGED-TYPE FLUORESCEIN)
22: UNCAGED-TYPE COMPOUND (UNCAGED-TYPE BIOTIN OR UNCAGED-TYPE FLUORESCEIN)
D: CONTAINER
F1, F2: LASER BEAM

## Claims

1. A cell separating method comprising:
a step of determining the characteristics of a cell labeled with a caged compound;
a step of activating the caged compound labeling a target cell by emitting light only to the target cell having a predetermined characteristic; and
a step of bringing cells into contact with a substance having an affinity with the activated caged compound and separating only the target cell from the cells based on the interaction between the substance and the activated caged compound.

2. A cell separating method comprising:
a step of determining the characteristics of a cell labeled with a caged compound;
a step of activating the caged compound labeling a non-target cell by emitting light only to the non-target cell not having a predetermined characteristic; and
a step of bringing cells into contact with a substance having an affinity with the activated caged compound and obtaining a target cell by removing only the non-target cell from the cells through separation based on the interaction between the substance and the activated caged compound.

3. The cell separating method according to Claim 1 or 2,
wherein, as the substance having an affinity with the activated caged compound, a substance having the affinity which is expressed when a photocleavable protecting group is cleaved from the caged compound is used.

4. A cell separating method comprising:
a step of determining the characteristics of a cell labeled with a caged compound;
a step of activating the caged compound labeling a non-target cell by emitting light only to the non-target cell having a predetermined characteristic; and
a step of bringing cells into contact with a substance having an affinity with a non-activated caged compound and separating only a target cell from the cells based on the interaction between the substance and the non-activated caged compound.

5. A cell separating method comprising:
a step of determining the characteristics of a cell labeled with a caged compound;
a step of activating the caged compound labeling a target cell by emitting light only to the target cell having a predetermined characteristic; and
a step of bringing cells into contact with a substance having an affinity with a non-activated caged compound and obtaining the target cell by removing only a non-target cell from the cells through separation based on the interaction between the substance and the non-activated caged compound.

6. The cell separating method according to Claim 4 or 5
wherein, as the substance having an affinity with the non-activated caged compound, a substance having the affinity which is lost when a photocleavable protecting group is cleaved from the caged compound is used.

7. The cell separating method according to Claim 3 or 6 further comprising a step of labeling a cell with a caged compound.

8. The cell separating method according to Claim 1 comprising:
a step of determining the characteristics of a cell labeled with caged biotin;
a step of activating the caged biotin labeling a target cell by emitting light only to the target cell having a predetermined characteristic; and
a step of bringing cells into contact with streptavidin and separating only the target cell from the cells based on the interaction between the streptavidin and the activated caged biotin.

9. The cell separating method according to Claim 1 comprising:
a step of determining the characteristics of a cell labeled with caged fluorescein;
a step of activating the caged fluorescein labeling a target cell by emitting light only to the target cell having a predetermined characteristic; and
a step of bringing cells into contact with an anti-fluorescein antibody and separating only the target cell from the cells based on the interaction between the antibody and the activated caged fluorescein.
